# EUROPÄISCHE PATENTANMELDUNG

(11) **EP 3 763 426 A1**
(43) Veröffentlichungstag der Anmeldung: **13.01.2021**
(21) Anmeldenummer: 19185927.1
(22) Anmeldetag: 12.07.2019
(51) Int. Cl.: B01D 3/00, C07C 263/20, C07C 275/60, C07D 251/34, C07D 273/04, C08G 18/73, C08G 18/76, C08G 18/78, C08G 18/02, C08G 18/10

(54) **DESTILLATION VON POLYISOCYANATEN**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Die Erfindung betrifft ein Verfahren zur Herstellung von monomerenarmen Polyisocyanaten umfassend die folgenden Schritte:
(i) Modifizierung mindestens eines monomeren Diisocyanats unter Erhalt eines Gemisches enthaltend mindestens ein Polyisocyanat und nicht umgesetztes monomeres Diisocyanat,
(ii) Trennung des in Schritt (i) erhaltenen Gemisches in mindestens einen gasförmigen, monomeres Diisocyanat enthaltenden Strom und einen flüssigen, an monomerem Diisocyanat abgereicherten Strom,
(iii) teilweise Kondensation des gasförmigen Stroms aus (ii) in mindestens einem Kondensator, wobei ein flüssiges Kondensat und ein nicht kondensierter Brüdenstrom erhalten werden,
(iv) Nachkondensation des in Schritt (iii) erhaltenen nicht kondensierten Brüdenstroms in mindestens einem Nachkondensator, wobei ein Nachkondensat und ein nicht kondensiertes Abgas erhalten werden und
(v) Zuführen des nicht kondensierten Abgases aus Schritt (iv) zur Saugseite einer Vakuumpumpe, dadurch gekennzeichnet, dass der mindestens eine Nachkondensator in Schritt (iv) bei einer Nachkondensatortemperatur betrieben wird, und der mindestens eine Kondensator in Schritt (iii) bei einer Kondensatortemperatur betrieben wird, wobei die Nachkondensatortemperatur um ≥ 1 bis ≤ 168 K niedriger liegt als die Kondensatortemperatur.

## Beschreibung

Die vorliegende Erfindung betrifft ein Verfahren zur Herstellung monomerenarmer Polyisocyanate sowie die danach erhältlichen Polyisocyanate und deren Verwendung als Ausgangsmaterialien zur Herstellung von Polymeren und die Verbundkörper enthaltend das Polymer.

Modifizierungsreaktionen aliphatischer und cycloaliphatischer Diisocyanate sind seit langem bekannt. Die dabei erhaltenen Polyisocyanate finden als Vernetzerkomponente in Beschichtungssystemen und Klebstoffen Verwendung. Üblich sind einerseits Modifizierungsreaktionen, in denen die Isocyanate mit sich selbst reagieren und die beispielsweise zur Bildung von Biureten, Isocyanuraten, Uretdionen oder Iminooxadiazindionen führen. Andererseits können die Isocyanate unter Bildung von Urethan-, Allophanat und/oder Harnstoffgruppen mit Poylolen oder Polyaminen umgesetzt und so oligomerisiert werden. Entscheidend ist die Bildung von höhermolekularen Addukten, die einen niedrigeren Dampfdruck aufweisen als die monomeren Diisocyanate selbst. Nicht umgesetztes Diisocyanat wird aus dem Reaktionsgemisch beispielsweise durch Dünnschichtdestillation abgetrennt und es verbleibt das Polyisocyanat als Sumpfprodukt, welches, falls gewünscht, mit Lösungsmittel verdünnt werden kann.

Die katalytische Herstellung von Polyisocyanaten und das destillative Entfernen von nicht umgesetztem Diisocyanat aus einer Polyisocyanat-Mischung ist beispielsweise in WO2008/068198A1 eingehend beschrieben. Das Entfernen des monomeren Diisocyanats erfolgt bevorzugt in einer mehrstufigen Destillation bei Temperaturen zwischen 90 und 220 °C im Vakuum. Der Druck sinkt vorzugsweise von Stufe zu Stufe und erreicht in der letzten Stufe 0,1 bis 10 hPa. Als Apparate kommen Flash-, Fallfilm-, Dünnschicht-, und/oder Kurzwegverdampfer zum Einsatz, wobei letztere wiederum bevorzugt in der letzten Stufe der Destillation zum Einsatz kommen, da sie konstruktionsbedingt einen geringen Druckverlust aufweisen und deshalb einen besonders niedrigen Druck ermöglichen. Auf diese Weise lässt sich das monomere Diisocyanat schonend entfernen. Es wird ausdrücklich darauf hingewiesen, die thermische Belastung möglichst gering zu halten, indem z.B. auf Zwischenbehälter und Vorlagebehälter verzichtet wird oder Sumpfvolumina klein und Rohrleitungen kurz gehalten werden. Das Destillat, also das monomere Diisocyanat wird bevorzugt in die Reaktion zurückgeführt und kann bei Bedarf einer zusätzlichen Behandlung zur Verbesserung der Farbzahl unterworfen werden, beispielsweise einer Filtration. Auf die Kondensation des Destillats und damit verbundene Probleme wird nicht eingegangen.

Auch DE102004038784A1 beschreibt das destillative Entfernen von nicht umgesetztem Diisocyanat aus einer Polyisocyanat-Mischung. Die Destillation erfolgt unter Einsatz mindestens eines Kurzwegverdampfers, gegebenenfalls in Kombination mit anderen Verdampfern. Als Prozessbedingungen wird ein Bereich von 5 mbar bis 10⁻⁴ mbar genannt und eine Verdampfertemperatur von 30 bis 230 °C. Auch auf die Innenkondensatortemperatur wird in dieser Schrift eingegangen und liegt im Bereich zwischen 5 und 150 °C. In einem Ausführungsbeispiel wird monomeres Methylendiisocyanat bei 0,05 mbar und einer Verdampfertemperatur von 177 °C destillativ abgetrennt und das verdampfte monomere Methylendiisocyanat bei 50 °C kondensiert.

EP1426393A2 beschreibt die Herstellung monomerenarmer uretdiongruppenhaltiger Polyisocyanate. In den Ausführungsbeispielen wird das destillative Entfernen von Hexamethylendiisocyanat aus der Polyisocyanatmischung beschrieben. Es erfolgt mittels eines Kurzwegverdampfers mit Vorverdampfer bei einem Druck von 0,1 bis 0,5 mbar und einer Heizmedium-Temperatur von 140 bis 150 °C. Nicht umgesetztes Monomer und der Katalysator werden auf diese Weise abgetrennt und das Destillat erneut zur Reaktion eingesetzt. Auf die hierzu erforderliche Kondensation des Destillats wird nicht eingegangen.

EP1241197A1 beschreibt die Herstellung monomerenarmer, Isocyanat-Prepolymere. Die Destillation der Rohware erfolgt bevorzugt in einem Kurzwegverdampfer bei einem Druck im Bereich von 0,1 bis 100 Pa und einer Kondensatortemperatur von 25 bis 75 °C.

EP1451239B1 beschreibt ein Verfahren zur Herstellung von MDI-Basierten Prepolymeren. Monomeres Diisocyanat wird destillativ in einer Reihe aus Verdampfern entfernt, wobei die Verdampfertemperatur in einem Bereich von 50 bis 210 °C liegt und die Kondensatortemperatur in einem Bereich von 15 bis 55 °C.

Zur Güte eines industriellen Herstellverfahrens gehört neben der Ausbeute und Qualität des Produkts auch die Zuverlässigkeit des Verfahrens, die einen stabilen Betrieb ohne technische Produktionsausfälle und damit einen möglichst wartungsarmen Betrieb der Produktionsanlagen ermöglicht. Bei der destillativen Abtrennung von monomerem Diisocyanat aus Polyisocyanaten im Vakuum ist es vorteilhaft, die thermische Belastung möglichst gering zu halten. Insbesondere für die Herstellung besonders monomerenarmer Polyisocyanate ist ein niedriger Druck im Destillationssystem erforderlich. Um diesen zu erreichen, muss die Kondensation des Destillats bei sehr niedrigen Temperaturen erfolgen, so dass das Vakuum nicht durch den Dampfdruck leichtsiedender Komponenten begrenzt wird. Die Destillation erfolgt, wie im oben zitierten Stand der Technik beschrieben, üblicherweise in einer Kombination aus Verdampfungsapparaten, die jeweils mit Kondensatoren ausgestattet sind. In der industriellen Praxis zeigte sich jedoch, dass bei der angestrebten geringen Betriebstemperatur der Kondensatoren regelmäßig Produktionsprobleme auftraten, die in der Folge zu Wartungsstillständen und damit Produktionsausfällen führten. So kam es verursacht durch die niedrige Kondensator-Temperatur zur Bildung von festen Ablagerungen am Kondensator. In der Folge stieg der Druckverlust zum Vakuumsystem und der erforderliche Unterdruck konnte nicht dauerhaft gehalten werden, so dass ein Stillstand zur Reinigung der Kondensatoren erforderlich wurde, um das Produkt weiterhin in der gewünschten monomerenarmen Qualität herzustellen. Versuche, die Probleme durch Variation der Kondensatortemperatur zu beheben, blieben erfolglos. Höhere Kondensatortemperaturen führten von vornherein zu Qualitätseinbußen, weil der erforderliche Unterdruck für die Destillation nicht mehr erreicht werden konnte. Darüber hinaus traten auch vermehrt Schäden an den Vakuumpumpen der Destillation auf.

Aufgabe der Erfindung war es also ein Verfahren zur Herstellung von monomerenarmen Polyisocyanaten zur Verfügung zu stellen, nach dem sich diese Polyisocyanate zuverlässig und in ausgezeichneter Qualität herstellen lassen.

Diese Aufgabe konnte gelöst werden durch ein Verfahren zur Herstellung von monomerenarmen Polyisocyanaten umfassend die folgenden Schritte:
(i) Modifizierung mindestens eines monomeren Diisocyanats unter Erhalt eines Gemisches enthaltend mindestens ein Polyisocyanat und nicht umgesetztes monomeres Diisocyanat,
(ii) Trennung des in Schritt (i) erhaltenen Gemisches in mindestens einen gasförmigen, monomeres Diisocyanat enthaltenden Strom und einen flüssigen, an monomerem Diisocyanat abgereicherten Strom,
(iii) teilweise Kondensation des gasförmigen Stroms aus (ii) in mindestens einem Kondensator, wobei ein flüssiges Kondensat und ein nicht kondensierter Brüdenstrom erhalten werden,
(iv) Nachkondensation des in Schritt (iii) erhaltenen nicht kondensierten Brüdenstroms in mindestens einem Nachkondensator, wobei ein Nachkondensat und ein nicht kondensiertes Abgas erhalten werden und
(v) Zuführen des nicht kondensierten Abgases aus Schritt (iv) zur Saugseite einer Vakuumpumpe,
dadurch gekennzeichnet, dass der mindestens eine Nachkondensator in Schritt (iv) bei einer Nachkondensatortemperatur betrieben wird, und der mindestens eine Kondensator in Schritt (iii) bei einer Kondensatortemperatur betrieben wird, wobei die Nachkondensatortemperatur um ≥ 1 bis ≤ 168 K niedriger liegt als die Kondensatortemperatur.

Ausdrücke wie "ein erster Verdampfer und ein zweiter Verdampfer" oder "erster Teil(strom) und ein zweiter Teil(strom)" oder "ein erster Kondensator und ein zweiter Kondensator" sind, sofern nicht ausdrücklich anders gesagt, stets als offene Formulierungen aufzufassen, die das Vorhandensein weiterer (dritter, vierter, ...) Verdampfer, Teil(ström)e oder Kondensatoren nicht ausschließen.

Bevorzugt bedeuten erfindungsgemäß die Ausdrücke "umfassend" oder "enthaltend", "im Wesentlichen bestehend aus" und besonders bevorzugt "bestehend aus".

Vorliegend steht "Polyisocyanat" für ein Isocyanat, welches durch Modifizierung aus einem Diisocyanat hergestellt wurde, wobei wenigstens zwei Diisocyanatmoleküle in das Polyisocyanat eingebaut wurden. Solche Polyisocyanate werden häufig auch als Lack-Polyisocyanate bezeichnet. "Polyisocyanat" steht also ausdrücklich nicht für ein Isocyanat, wie es direkt aus der Phosgenierungsreaktion eines Di- oder Polyamins erhalten wird.

Unter "Kondensatortemperatur", "Nachkondensatortemperatur" bzw. "Kühlfallentemperatur" ist vorliegend jeweils die Vorlauftemperatur des zugehörigen Kühlmediums zu verstehen, sofern in der Beschreibung nichts anderes angegeben ist.

"Mindestens ein Diisocyanat", wie hierin verwendet, bezieht sich auf 1 oder mehr, beispielsweise 2, 3, 4, 5, 6, 7, 8, 9 oder mehr Diisocyanate. Im Zusammenhang mit Bestandteilen der hierin beschriebenen Verbindungen bezieht sich diese Angabe nicht auf die absolute Menge an Molekülen, sondern auf die Art des Bestandteils. "Mindestens ein Diisocyanat" bedeutet daher beispielsweise, dass nur eine Art von Diisocyanat oder mehrere verschiedene Arten von Diisocyanaten, ohne Angaben über die Menge der einzelnen Verbindungen zu machen, enthalten sein können.

### Schritt (i)

Die Modifizierung des Diisocyanats in Schritt (i) erfolgt nach an sich bekannten Verfahren und wird auch als Modifikation oder Oligomerisierung bezeichnet. Das Isocyanat reagiert dabei unter geeigneten Reaktionsbedingungen entweder mit sich selbst oder aber mit weiteren Verbindungen wie beispielsweise Polyolen, Polythiolen oder Polyaminen. Bevorzugt erfolgt die Umsetzung in Anwesenheit eines Katalysators und wird beim Erreichen eines bestimmten Umsetzungsgrades durch Desaktivierung dieses Katalysators gestoppt. Die Desaktivierung kann auf verschiedene Arten erfolgen. Bevorzugt erfolgt sie durch Zugabe einer Verbindung, die den Katalysator desaktiviert. Manche Katalysatoren lassen sich auch thermisch Desaktivieren oder aus dem Reaktionsgemisch entfernen, um die Reaktion zu stoppen.

Als monomere Diisocyanate für die Modifizerung eignen sich zunächst alle industriell verfügbaren aliphatischen, cycloaliphatischen, araliphatischen oder aromatischen Diisocyanate.

Geeignete aliphatische, cycloaliphatische, araliphatische oder aromatische Diisocyanate sind beispielsweise ausgewählt aus der Gruppe bestehend aus 1,4-Diisocyanatobutan, 1,5-Diisocyanatopentan (PDI), 1,6-Diisocyanatohexan (HDI), 2-Methyl-1,5-diisocyanatopentan, 1,5-Diisocyanato-2,2-dimethylpentan, 2,2,4- bzw. 2,4,4-Trimethyl-1,6-diisocyanatohexan, 1,10-Diisocyanatodecan, 1,3- und 1,4-Diisocyanatocyclohexan, 2,4- und 2,6-Diisocyanato-1-methylcyclohexan, 1,3- und 1,4-Bis-(isocyanatomethyl)-cyclohexan, 1-Isocyanato-3,3,5-trimethyl-5-isocyanatomethylcyclohexan (Isophorondiisocyanat, IPDI), 4,4'-Diisocyanatodicyclohexylmethan, 2,4'-Diisocyanatodicyclohexylmethan, 1-Isocyanato-1-methyl-4(3)isocyanato-methylcyclohexan, Bis-(isocyanatomethyl)-norbornan, 1,3- und 1,4-Bis(isocyanatomethyl)benzol (XDI), 1,3- und 1,4-Bis-(2-isocyanato-prop-2-yl)-benzol (TMXDI), 2,4- und 2,6-Toluylendiisocyanat (TDI), 2,4'- und 4,4'-Diisocyanatodiphenylmethan (MDI), 1,5-Diisocyanatonaphthalin, 1,3- und 1,4-Phenylendiisocyanat, oder beliebige Gemische solcher Diisocyanate.

Besonders bevorzugt sind die monomeren Diisocyanate ausgewählt aus der Gruppe bestehend aus 1,5-Diisocyanatopentan (PDI), 1,6-Diisocyanatohexan (HDI), 1-Isocyanato-3,3,5-trimethyl-5-isocyanatomethylcyclohexan (Isophorondiisocyanat, IPDI), 4,4'-Diisocyanatodicyclohexylmethan, 2,4'-Diisocyanatodicyclohexylmethan, Bis-(isocyanatomethyl)-norbornan, 1,3- und 1,4-Bis(isocyanatomethyl)benzol (XDI), Toluylendiisocyanat (TDI), 2,4'- und 4,4'-Diisocyanatodiphenylmethan (MDI) oder beliebige Gemische solcher Diisocyanate.

Ganz besonders bevorzugt sind die monomeren Diisocyanate ausgewählt aus der Gruppe bestehend aus 1,5-Diisocyanatopentan (PDI), 1,6-Diisocyanatohexan (HDI) und 1-Isocyanato-3,3,5-trimethyl-5-isocyanatomethylcyclohexan (Isophorondiisocyanat, IPDI) oder Gemische dieser Isocyanate untereinander oder mit TDI.

Am meisten bevorzugt sind die monomeren Diisocyanate ausgewählt aus der Gruppe bestehend aus PDI und HDI.

Bei den Polyisocyanaten, die durch die Modifizierung dieser Isocyanate entstehen, handelt es sich beispielsweise um Biuretpolyisocyanate, Isocyanuratpolyisocyanate, Uretdionpolyisocyanate, Urethanpolyisocyanate, Allophanatpolyisocyanate, Oxadiazintrionpolyisocyanate, Iminooxadiazindionpolyisocyanate, Carbodiimidpolyisocyanate und/oder Harnstoffpolyisocyanate. Dabei sind auch Mischformen möglich, also solche, die beispielsweise Allophanatgruppen und Isocyanuratgruppen oder Allophanatgruppen und Urethangruppen enthalten. Diese können der einen oder der anderen Gruppe zugeordnet werden. Bevorzugt werden sie derjenigen Gruppe zugeordnet, die häufiger im Polyisocyanat vertreten ist.

In einer weiteren vorteilhaften Ausführung des erfindungsgemäßen Verfahrens ist die Modifizierung in Schritt (i) eine intermolekulare Reaktion zwischen Isocyanat-Gruppen, bevorzugt in Gegenwart eines Katalysators, insbesondere in Gegenwart eines basischen Katalysators, unter Bildung von Uretdionpolyisocyanaten, Isocyanuratpolyisocyanaten oder Iminooxadiazindionpolyisocyanaten und/oder eine Reaktion von Isocyanaten mit bereits gebildeten Urethangruppen unter Bildung von Allophanatpolyioscyanaten.

Besonders bevorzugt handelt es sich um die Trimerisierung zu Isocyanuratpolyisocyanaten beziehungsweise Iminooxadiazindionpolyisocyanaten oder die Dimerisierung zu Uretdionpolyioscyanaten. Ganz besonders bevorzugt handelt es sich um die Trimerisierung zu Isocyanuratpolyisocyanaten beziehungsweise Iminooxadiazindionpolyisocyanaten.

In einer weiteren bevorzugten Ausführungsform der vorliegenden Erfindung ist das Polyisocyanat ausgewählt aus der Gruppe bestehend aus Isocyanuratpolyisocyanaten, Biuretpolyisocyanaten, Iminooxadiazindionpolyisocyanaten, Urethanpolyisocyanaten und Allophanatpolyisocyanaten, besonders bevorzugt aus der Gruppe bestehend aus Isocyanuratpolyisocyanaten, Biuretpolyisocyanaten und Iminooxadiazindionpolyisocyanaten. Gerade diese Isocyanate neigen zu den eingangs beschriebenen Problemen bei der destillativen Entfernung der nicht umgesetzten monomeren Diisocyanate, weshalb sie besonders für das vorliegende Verfahren geeignet sind.

### Schritt (ii)

Die Trennung des in Schritt (i) erhaltenen Gemisches in einen gasförmigen, monomeres Diisocyanat enthaltenden Strom und einen flüssigen, an monomerem Diisocyanat abgereicherten Strom erfolgt vorzugsweise in mindestens einem Verdampfer, der in der Regel bei einem Druck im Bereich von 0,005 mbar bis 400 mbar, bevorzugt 0,005 bis 100 mbar, besonders bevorzugt 0,005 bis 10 mbar und ganz besonders bevorzugt 0,005 bis 2 mbar und einer Temperatur im Bereich von 90 bis 205 °C, bevorzugt im Bereich von 100 bis 185 °C betrieben wird, wobei der flüssige, an monomerem Diisocyanat abgereicherte Strom als Sumpfprodukt erhalten wird.

Vorliegend bezieht sich der Begriff "abgereichert" auf den entsprechenden Gehalt der Komponente im Ausgangsfluid, aus dem das Fluid gebildet wurde. Das Fluid ist abgereichert, wenn es einen niedrigeren Gehalt, bevorzugt höchstens den 0,9-fachen Gehalt der entsprechenden Komponente enthält. Die Gehalte sind dabei jeweils als Massengehalte bezogen auf die Gesamtmasse des jeweiligen Fluids.

Vorliegend enthält der abgereicherte Strom besonders bevorzugt höchstens den 0,5-fachen Gehalt, ganz besonders bevorzugt höchstens den 0,1-fachen und am meisten bevorzugt höchstens den 0,02-fachen Gehalt des monomeren Diisocyanats, bezogen auf den Gehalt in dem in Schritt (i) erhaltenen Gemisch. In einer bevorzugten Ausführungsform mit mehrstufiger Verdampfung enthält in der letzten Stufe erhaltene abgereicherte Strom bevorzugt höchstens den 0,1-fachen, besonders bevorzugt höchstens den 0,01-fachen und ganz besonders bevorzugt höchstens den 0,002-fachen Gehalt des monomeren Diisocyanats bezogen auf den Gehalt im Zulaufstrom der ersten Stufe. Eine Abreicherung auf weniger als den 0,0005-fachen Gehalt, vorzugsweise weniger als den 0,001-fachen Gehalt des monomeren Diisocyanats ist in der Regel nicht erforderlich und deshalb aus wirtschaftlichen Gründen auch nicht sinnvoll.

Als Verdampfer eignen sich vorzugsweise Fallfilmverdampfer, Dünnschichtverdampfer oder Kurzwegverdampfer. In einer bevorzugten Ausführungsform des erfindungsgemäßen Verfahrens wird die Trennung mehrstufig, vorzugsweise in 2 bis 5 Stufen, besonders bevorzugt in 2 bis 4 Stufen, ganz besonders bevorzugt in 3 bis 4 Stufen und am meisten bevorzugt in 3 Stufen ausgeführt. Unter der mehrstufigen Ausführung ist zu verstehen, dass mehrere Verdampfer mit jeweils wenigstens einem Zulauf und wenigstens einem Sumpfablauf in Reihe eingesetzt werden, wobei jeweils der Sumpfablauf eines Verdampfers als Zulauf des nächsten Verdampfers dient. Es ist auch denkbar, eine oder mehrere dieser Verdampfungsstufen in mehreren parallel betriebenen Verdampfern durchzuführen, was jedoch mit einem erhöhten apparativen Aufwand einhergeht.

In der bevorzugten Ausführungsform mit einer mehrstufig ausgeführten Trennung des in Schritt (i) erhaltenen Gemisches in Schritt (ii) ist es vorteilhaft, den Druck von Stufe zu Stufe zu senken, wobei zumindest die letzte Stufe bevorzugt bei einem Druck zwischen 0,005 und 10 mbar, besonders bevorzugt zwischen 0,005 und 2 mbar betrieben wird. Um eine besonders schonende Trennung durchzuführen liegt der Druck auch in der ersten Stufe im Bereich von 2 bis 100 mbar.

Beim flüssigen, an monomerem Diisocyanat abgereicherten Strom, der in Schritt (ii) erhalten wird, handelt es sich um das gewünschte monomerenarme Polyisocyanat, welches weniger als 0,5 Gew.-%, bevorzugt weniger als 0,3 Gew.-%, besonders bevorzugt weniger als 0,2 Gew.-% und ganz besonders bevorzugt weniger als 0,1 Gew.-% des monomeren Diisocyanats bezogen auf die Gesamtmasse des Stroms enthält. In der bevorzugten Ausführungsform mit einer mehrstufig ausgeführten Verdampfung in Schritt (ii) fällt das Produkt als Sumpfstrom der letzten Verdampfungsstufe an.

Bevorzugt wird in der letzten Verdampfungsstufe ein Kurzwegverdampfer eingesetzt. Kurzwegverdampfer zeichnen sich durch einen innenliegenden Kondensator aus. Die teilweise Kondensation des gasförmigen, Diisocyanat enthaltenden Stroms (Schritt (iii)) findet also in dieser bevorzugten Ausführungsform bereits im Kurzwegverdampfer statt. Dadurch reduziert sich der Gasvolumenstrom in die nachgeschalteten Apparate und der Druckverlust wird minimiert, was ein besonders niedriges Vakuum und somit eine besonders schonende Trennung ermöglicht.

### Schritt (iii)

Die teilweise Kondensation des gasförmigen, Diisocyanat enthaltenden Stroms erfolgt in mindestens einem Kondensator vorzugsweise bei einer Kondensatortemperatur im Bereich von 16 bis 135 °C. Die am besten geeignete Kondensatortemperatur kann vom Fachmann in Abhängigkeit der vorliegenden Randbedingungen wie beispielsweise des abzutrennenden monomeren Diisocyanats, der gewählten Druckstufe und des Kondensators sowie dessen Wärmeübertragungsfläche ermittelt werden. Besonderes bevorzugt erfolgt die Kondensation bei einer Kondensatortemperatur im Bereich von 16 bis 40 °C, ganz besonders bevorzugt im Bereich von 18 bis 35 °C. Als Apparate für die Kondensation eignen sich Wärmetauscher, vorzugsweise Rohrbündelwärmetauscher, wobei es besonders bevorzugt ist, die Kondensation im Mantelraum durchzuführen, während die Rohre mit einem Wärmeträgermedium durchströmt werden, welches die freigesetzte Kondensationswärme abtransportiert. Bei Verwendung eines Kurzwegverdampfers erfolgt die teilweise Kondensation bereits am innenliegenden Kondensator des Kurzwegverdampfers selbst.

Als Wärmeträgermedium für den Betrieb des mindestens einen Kondensators in der teilweisen Kondensation des gasförmigen Gemisches in Schritt (iii) eignen sich beispielsweise Wasser, Alkohol-Wasser-Gemische, Salz-Wasser-Lösungen, Thermalöle oder organische Lösungsmittel wie zum Beispiel Chlorbenzol. Bevorzugt werden Wasser, Wasser-Alkohol-Gemische oder Salz-Wasser-Lösungen verwendet, besonders bevorzugt wird Wasser als Wärmeträgermedium eingesetzt.

In einer alternativen Ausführungsform des erfindungsgemäßen Verfahrens erfolgt die teilweise Kondensation des gasförmigen, Diisocyanat enthaltenden Stroms in Schritt (iii) in einem Luftkühler. Dabei wird durch einen Ventilator Umgebungsluft an der Außenseite eines gegebenenfalls mit Kühlrippen versehenen Rohrbündels entlang geleitet, um dieses zu kühlen. Die Kondensation erfolgt innerhalb der Rohre, an deren Ende das flüssige Kondensat sowie der nicht kondensierte Brüdenstrom zunächst gemeinsam austreten und dann getrennt werden. In diesem Spezialfall ist unter der Kondensatortemperatur die Austrittstemperatur des nicht kondensierten Brüdenstroms zu verstehen.

### Schritt (iv)

Die Nachkondensation des nicht kondensierten Brüdenstroms aus Schritt (iii) erfolgt in mindestens einem Nachkondensator vorzugsweise bei einer Nachkondensatortemperatur im Bereich von -33 bis 130 °C. Die am besten geeignete Nachkondensatortemperatur kann vom Fachmann in Abhängigkeit der vorliegenden Randbedingungen wie beispielsweise des abzutrennenden monomeren Diisocyanats, der gewählten Druckstufe und des Nachkondensators sowie dessen Wärmeübertragungsfläche ermittelt werden. Besonderes bevorzugt erfolgt die Nachkondenstation bei einer Nachkondensatortemperatur im Bereich von -20 bis 25 °C, ganz besonders bevorzugt im Bereich von -10 bis 17 °C. Dabei ist die Nachkondensatortemperatur so gewählt, dass sie um 1 bis 168 K, bevorzugt um 5 bis 50 K und besonders bevorzugt um 10 bis 30 K unterhalb der Kondensatortemperatur des Kondensators in Schritt (iii) liegt.

Bei Bedarf, beispielsweise aufgrund von Veränderungen der Mengenströme oder der Zusammensetzung des Brüdenstroms, ist es möglich, die Kondensatortemperatur und/oder die Nachkondensatortemperatur während der Durchführung des erfindungsgemäßen Verfahrens unter Einhaltung der zuvor genannten Differenz zwischen Kondensator- und Nachkondensatortemperatur zu variieren.

Als Apparate für die Nachkondensation eignen sich Wärmetauscher, vorzugsweise Rohrbündelwärmetauscher, wobei es besonders bevorzugt ist, die Nachkondensation im Mantelraum durchzuführen, während die Rohre mit einem Wärmeträgermedium durchströmt werden, welches die freigesetzte Nachkondensationswärme abtransportiert.

In einigen Fällen kann es bei tiefen Temperaturen zum Erstarren von Anteilen des Nachkondensats an den kalten Oberflächen des Nachkondensators kommen. Um ein Verstopfen durch die erstarrten Ablagerungen zu verhindern, kann von Zeit zu Zeit ein Abschmelzvorgang bei erhöhter Nachkondensatortemperatur erfolgen.

Als Wärmeträgermedium für den Betrieb des mindestens einen Nachkondensators in der Nachkondensation des nicht kondensierten Brüdenstroms aus Schritt (iii) in Schritt (iv) eignen sich beispielsweise Wasser, Alkohol-Wasser-Gemische, Salz-Wasser-Lösungen, Thermalöle oder organische Lösungsmittel wie zum Beispiel Chlorbenzol. Bevorzugt werden Wasser, Wasser-Alkohol-Gemische oder Salz-Wasser-Lösungen verwendet, besonders bevorzugt werden Salz-Wasser-Lösungen oder Wasser-Alkohol-Gemische verwendet. Das Wärmeträgermedium ist so zu wählen, dass dessen Erstarrungspunkt unterhalb der gewünschten Nachkondensatortemperatur des Nachkondensators liegt.

### Schritt (v)

Das in Schritt (iv) erhaltene, nicht kondensierte Abgas aus dem Nachkondensator wird der Saugseite einer Vakuumpumpe zugeführt. Hierzu ist der Gasraum des Nachkondensators mit der Saugseite der Vakuumpumpe fluidisch verbunden.

In einer weiteren bevorzugten Ausführungsform der Erfindung wird zwischen den Schritten (iv) und (v) ein weiterer Schritt (iv-a) durchgeführt, in dem der nicht kondensierte Abgasstrom aus dem Nachkondensator in einem weiteren Wärmetauscher, einer Kühlfalle mit einer Kühlfallentemperatur, die mindestens 1 K, bevorzugt mindestens 5 K und besonders bevorzugt mindestens 10 K unterhalb der Nachkondensatortemperatur liegt, weiter abgekühlt wird. Diese Kühlfalle ist fluidisch einerseits mit dem Abgasauslass von mindestens einem Nachkondensator und andererseits mit der Saugseite mindestens einer Vakuumpumpe verbunden. Die Kühlfalle kann beispielsweise mit einem Kühlmedium bei einer Temperatur im Bereich von -10 bis -200 °C betrieben werden. Bevorzugt liegt die Temperatur des Kühlmediums im Bereich von -15 bis -60 °C und besonders bevorzugt im Bereich von -16 bis -30 °C. In jedem Falle liegt die Temperatur des Kühlmediums mindestens 1 K, bevorzugt mindestens 5 K und besonders bevorzugt mindestens 10 K unterhalb der Nachkondensatortemperatur. Alternativ kann die Kühlung beispielsweise auch über mindestens ein Peltier-Element erfolgen, sofern die Betriebstemperatur der Kühlfalle, also in diesem Spezialfall die Temperatur zumindest eines Teils der Wärmeübertragungsfläche in den zuvor genannten Bereichen für die Temperatur des Kühlmediums liegt. Im Falle einer mehrstufigen Ausführung der Destillationsvorrichtung ist die Kühlfalle vorzugsweise so angeordnet, dass sie zumindest von einem Teil des Abgases aus der ersten Destillationsstufe durchströmt wird und diesen abkühlt, bevor es die Saugseite der zugehörigen Vakuumpumpe erreicht. Besonders bevorzugt durchläuft der gesamte Abgasstrom die Kühlfalle vor Eintritt in die am weitesten stromabwärts gelegene Vakuumpumpe.

Die Vakuumpumpe kann ihrerseits Teil eines mehrstufigen Vakuum-Systems sein. Eine solche Anordnung ist in den Ausführungsbeispielen näher beschrieben.

Die im vorherigen beschriebenen Schritte (i), (ii), (iii), (iv), (iv-a) und (v) können bei Bedarf ergänzt werden durch weitere Prozessschritte.

Beispielhaft sei hier erwähnt, dass an verschiedenen Stellen des erfindungsgemäßen Verfahrens, z.B. am Brüden-Austritt des Verdampfers, Tropfenabscheider installiert sein können. Ein weiteres Beispiel ist die Verwendung von Filtern, z.B. vor dem Eintritt in eine Vakuumpumpe oder am Sumpfaustrag des letzten Verdampfers, also im Produktstrom.

In einer weiteren bevorzugten Ausführungsform des Verfahrens wird das flüssige Kondensat aus Schritt (iii) zumindest teilweise, gegebenenfalls nach weiteren Reinigungsschritten, in Schritt (i) des Verfahrens, also die Modifizierung eines monomeren Diisocyanats zurückgeführt. Auf diese Weise wird Abfall vermieden, und das Verfahren kann besonders wirtschaftlich betrieben werden. Optional kann zusätzlich zum flüssigen Kondensat aus Schritt (iii) oder statt des flüssigen Kondensats aus Schritt (iii) auch das Nachkondensat zumindest teilweise, gegebenenfalls nach weiteren Reinigungsschritten, in Schritt (i) zurückgeführt werden. Sofern das flüssige Kondensat und das Nachkondensat zurückgeführt werden, gibt es verschiedene Ausführungsformen. So ist es beispielsweise möglich, das flüssige Kondensat aus der Kondensation (Schritt (iii)) und das Nachkondensat aus Schritt (iv) getrennt oder, nach deren Zusammenführung zu einem vereinigten Kondensat, gemeinsam in Schritt (i) zurückzuführen. Sofern ein weiterer Reinigungsschritt des flüssigen Kondensats und/oder des Nachkondensats erfolgt, wie beispielsweise eine Filtration oder Destillation, kann dieser entweder nur am Kondensat, nur am Nachkondensat, am Kondensat und Nachkondensat jeweils separat oder am vereinigten Kondensat erfolgen.

Eine weitere bevorzugte Ausführungsform der Erfindung ist ein Verfahren zur Herstellung von monomerenarmen Polyisocyanaten umfassend die folgenden Schritte:
(i) Modifizierung mindestens eines monomeren Diisocyanats unter Erhalt eines Gemisches enthaltend mindestens ein Polyisocyanat und nicht umgesetztes monomeres Diisocyanat,
(ii) Trennung des in Schritt (i) erhaltenen Gemisches in mindestens einen gasförmigen, monomeres Diisocyanat enthaltenden Strom und einen flüssigen, an monomerem Diisocyanat abgereicherten Strom,
(iii) teilweise Kondensation des gasförmigen Stroms aus (ii) in mindestens einem Kondensator, wobei ein flüssiges Kondensat und ein nicht kondensierter Brüdenstrom erhalten werden,
(iv) Nachkondensation des in Schritt (iii) erhaltenen nicht kondensierten Brüdenstroms in mindestens einem Nachkondensator, wobei ein Nachkondensat und ein nicht kondensiertes Abgas erhalten werden und
(v) Zuführen des nicht kondensierten Abgases aus Schritt (iv) zur Saugseite einer Vakuumpumpe,
dadurch gekennzeichnet, dass der mindestens eine Nachkondensator in Schritt (iv) bei einer Nachkondensatortemperatur betrieben wird, und der mindestens eine Kondensator in Schritt (iii) bei einer Kondensatortemperatur betrieben wird, wobei die Nachkondensatortemperatur um ≥ 1 bis ≤ 168 K niedriger liegt als die Kondensatortemperatur und dass das flüssige Kondensat aus Schritt (iii) zumindest teilweise, gegebenenfalls nach weiteren Reinigungsschritten, in Schritt (i) des Verfahrens, also die Modifizierung des mindestens einen monomeren Diisocyanats, zurückgeführt wird.

Alternativ ist eine weitere bevorzugte Ausführungsform der Erfindung ein Verfahren zur Herstellung von monomerenarmen Polyisocyanaten umfassend die folgenden Schritte:
(i) Modifizierung mindestens eines monomeren Diisocyanats unter Erhalt eines Gemisches enthaltend mindestens ein Polyisocyanat und nicht umgesetztes monomeres Diisocyanat,
(ii) Trennung des in Schritt (i) erhaltenen Gemisches in mindestens einen gasförmigen, monomeres Diisocyanat enthaltenden Strom und einen flüssigen, an monomerem Diisocyanat abgereicherten Strom,
(iii) teilweise Kondensation des gasförmigen Stroms aus (ii) in mindestens einem Kondensator, der bei einer Kondensatortemperatur von 16 bis 135 °C, bevorzugt 16 bis 40 °C und besonders bevorzugt 18 bis 35 °C betrieben wird, wobei ein flüssiges Kondensat und ein nicht kondensierter Brüdenstrom erhalten werden,
(iv) Nachkondensation des in Schritt (iii) erhaltenen nicht kondensierten Brüdenstroms in mindestens einem Nachkondensator, der bei einer Nachkondensatortemperatur von -33 bis 130 °C, bevorzugt -20 bis 25 °C und besonders bevorzugt -10 bis 17 °C betrieben wird, wobei ein Nachkondensat und ein nicht kondensiertes Abgas erhalten werden und
(v) Zuführen des nicht kondensierten Abgases aus Schritt (iv) zur Saugseite einer Vakuumpumpe,
dadurch gekennzeichnet, dass die Nachkondensatortemperatur des mindestens einen Nachkondensators in Schritt (iv) um ≥ 1 bis ≤ 168 K, bevorzugt 5 bis 50 K und besonders bevorzugt 10 bis 30 K niedriger liegt als die Kondensatortemperatur des mindestens einen Kondensators in Schritt (iii).

Eine besonders bevorzugte Ausführungsform der Erfindung ist ein Verfahren zur Herstellung von monomerenarmen Polyisocyanaten umfassend die folgenden Schritte:
(i) Modifizierung mindestens eines monomeren Diisocyanats unter Erhalt eines Gemisches enthaltend mindestens ein Polyisocyanat und nicht umgesetztes monomeres Diisocyanat,
(ii) Trennung des in Schritt (i) erhaltenen Gemisches in mindestens einen gasförmigen, monomeres Diisocyanat enthaltenden Strom und einen flüssigen, an monomerem Diisocyanat abgereicherten Strom,
(iii) teilweise Kondensation des gasförmigen Stroms aus (ii) in mindestens einem Kondensator, der bei einer Kondensatortemperatur von 16 bis 135 °C, bevorzugt 15 bis 40 °C und besonders bevorzugt 18 bis 35 °C betrieben wird, wobei ein flüssiges Kondensat und ein nicht kondensierter Brüdenstrom erhalten werden,
(iv) Nachkondensation des in Schritt (iii) erhaltenen nicht kondensierten Brüdenstroms in mindestens einem Nachkondensator, der bei einer Nachkondensatortemperatur von -33 bis 130 °C, bevorzugt -20 bis 25 °C und besonders bevorzugt -10 bis 17 °C betrieben wird, wobei ein Nachkondensat und ein nicht kondensiertes Abgas erhalten werden und
(v) Zuführen des nicht kondensierten Abgases aus Schritt (iv) zur Saugseite einer Vakuumpumpe,
dadurch gekennzeichnet, dass die Nachkondensatortemperatur des mindestens einen Nachkondensators in Schritt (iv) um ≥ 1 bis ≤ 168 K, bevorzugt 5 bis 50 K und besonders bevorzugt 10 bis 30 K niedriger liegt als die Kondensatortemperatur des mindestens einen Kondensators in Schritt (iii) und dass, das flüssige Kondensat aus Schritt (iii) zumindest teilweise und, gegebenenfalls nach weiteren Reinigungsschritten, in Schritt (i) des Verfahrens, also die Modifizierung des mindestens einen monomeren Diisocyanats, zurückgeführt wird.

Gegenstand der Erfindung ist auch die Verwendung der nach dem erfindungsgemäßen Verfahren hergestellten Polyisocyanate als Ausgangsmaterialien zur Herstellung von Polymeren wie geschäumten Kunststoffen, Polyurethan-Lacken, Beschichtungsmitteln, Klebstoffen oder Zuschlagstoffen.

Insbesondere die Verwendung zur Herstellung von Ein- und Zweikomponenten-Polyurethanlacken sind die erfindungsgemäß hergestellten Polyisocyanate geeignet.

Bei der Verwendung als Vernetzerkomponente in 2K-Beschichtungen werden die erfindungsgemäßen Polyisocyanate in der Regel mit OH-und/oder NH Komponenten kombiniert, wie sie aus 2K-Polyurethansystemen an sich bekannt sind, so z. B. hydroxyfunktionellen Polyestern, Polyacrylaten, Polycarbonaten, Polyethern, Polyurethanen sowie polyfunktionellen Aminen. Sie können aber auch einkomponentig zur Herstellung (anteilig) feuchtigkeitshärtender Kunststoffe und Beschichtungen verwendet werden.

Ein weiterer Gegenstand der Erfindung ist ein Verbundkörper umfassend wenigstens ein erfindungsgemäßes Polymer in direktem Kontakt mit wenigstens einem Substrat bestehend aus Metall, Kunststoff, Holz oder deren Mischungen.

### Beispiele

Alle Prozentangaben beziehen sich, soweit nichts Anderslautendes vermerkt ist, auf das Gewicht.

Die Bestimmung der NCO-Gehalte erfolgte titrimetrisch nach DIN EN ISO 11909:2007-05.

Die Rest-Monomeren Gehalte wurden nach DIN EN ISO 10283:2007-11 gaschromatographisch mit internem Standard gemessen.

### Rohprodukt A:

Isocyanurat-Polyisocyanat hergestellt in an sich bekannter Weise durch katalytische Trimerisierung von Hexamethylendiisocyanat. Der Restmonomergehalt nach Abstoppen der Reaktion betrug ca. 77% Hexamethylendiisocyanat bezogen auf das gesamte Reaktionsgemisch.

### Rohprodukt B:

Isocyanurat-Polyisocyanat hergestellt in an sich bekannter Weise durch katalytische Trimerisierung von Pentamethylendiisocyanat. Der Restmonomergehalt nach Abstoppen der Reaktion betrug ca. 50% Pentamethylendiisocyanat bezogen auf das gesamte Reaktionsgemisch.

### Rohprodukt C:

Iminooxadiazindion-Polyisocyanat hergestellt in an sich bekannter Weise durch katalytische Trimerisierung von Hexamethylendiisocyanat. Der Restmonomergehalt nach Abstoppen der Reaktion betrug ca. 45% Hexamethylendiisocyanat bezogen auf das gesamte Reaktionsgemisch.

### Rohprodukt D:

Isocyanat-terminiertes Prepolymer hergestellt in an sich bekannter Weise durch Umsetzung eines Polyether-Polyols mit überschüssigem Toluylendiisocyanat. Der Restmonomergehalt nach der Reaktion betrug ca. 38% Toluylendiisocyanat bezogen auf das gesamte Reaktionsgemisch.

### Rohprodukt E:

Allophanatgruppen und Isocyanuratgruppen enthaltendes Polyisocyanat hergestellt in an sich bekannter Weise durch katalytische Umsetzung von Hexamethylendiisocyanat mit einem einwertigen Alkohol in Gegenwart eines Trimerisierungskatalysators. Der Restmonomergehalt nach Abstoppen der Reaktion betrug ca. 40% Hexamethylendiisocyanat bezogen auf das gesamte Reaktionsgemisch.

### Vergleichsbeispiele 1a-e (nicht erfindungsgemäß):

Die Rohprodukte A-E wurden jeweils in einer dreistufigen Vakuum-Destillationsvorrichtung kontinuierlich destilliert, um verbliebenes Monomer aus dem Polyisocyanat abzutrennen. Auch das Vakuumsystem war mehrstufig ausgeführt, so dass für jede Destillationsstufe eine Vakuumpumpe vorhanden war, wobei der druckseitige Abgasstrom aus der Vakuumpumpe der dritten Destillationsstufe zusammen mit dem nicht kondensierten Abgas der zweiten Destillationsstufe zur Saugseite der Vakuumpumpe der zweiten Destillationsstufe geleitet wurden. Analog wurde der druckseitige Abgasstrom der Vakuumpumpe der zweiten Destillationsstufe zusammen mit dem nicht kondensierten Abgas der ersten Destillationsstufe zur Saugseite der Vakuumpumpe der ersten Destillationsstufe geleitet. Der druckseitige Abgasstrom der Vakuumpumpe der ersten Destillationsstufe wurde über das Abgassystem der Anlage entsorgt.

Als erste Destillationsstufe wurde ein Fallfilmverdampfer verwendet, der mit Dampf beheizt wurde. Der Druck in dieser ersten Verdampfungsstufe lag bei 25 mbar. Die Brüden aus dem Fallfilmverdampfer wurden in einem Kondensator kondensiert, der mit Kühlwasser betrieben wurde. Die Temperatur des Kühlwassers im Vorlauf betrug 27 °C. Nicht kondensierte Anteile wurden über das Vakuumsystem aus der Destillationsvorrichtung entfernt.

Der nicht verdampfte Sumpfstrom wurde als Zulauf in einen zweiten Fallfilmverdampfer geleitet, wo weiteres Monomer aus dem nun vorgereinigten Polyisocyanat verdampft wurde. Auch diese zweite Destillationsstufe war als Fallfilmverdampfer ausgeführt, der bei einem Druck von 7 mbar betrieben und ebenfalls mittels Dampf beheizt wurde. Die Brüden wurden wiederum in einem Kondensator kondensiert, der mit Kühlwasser betrieben wurde. Die Temperatur des Kühlwassers im Vorlauf betrug ebenfalls 27 °C. Nicht kondensierte Anteile wurden über das Vakuumsystem aus der Destillationsvorrichtung entfernt.

Der nicht verdampfte Sumpfstrom aus dieser zweiten Destillationsstufe wurde wiederum als Zulauf in die letzte Destillationsstufe geleitet. Diese letzte Destillationsstufe war als Kurzwegverdampfer mit innenliegendem Kondensator ausgeführt, wobei die Heizflächen mit Dampf beheizt wurden und der innenliegende Kondensator mit Kühlwasser betrieben wurde, welches wiederum eine Vorlauftemperatur von 27 °C aufwies. Der Druck im Kurzwegverdampfer betrug 1,4 mbar. Nicht kondensierte Anteile der Brüden wurden über das Vakuumsystem aus der Destillationsvorrichtung entfernt. Das Sumpfprodukt wurde abgekühlt und mittels Gaschromatographie analysiert.

Kurzfristig konnten Restmonomeren-Gehalte von <0,1 % erreicht werden, doch erreichten die Vakuumpumpen nur sehr geringe Standzeiten von 7-18 Tagen, so dass der Betrieb immer wieder unterbrochen werden musste, um diese instandzusetzen oder auszutauschen. Darüber hinaus kam es beim Ausfall der Vakuumpumpen in einigen Fällen auch zum Durchschlag von Sumpfprodukt mit höheren Restmonomergehalten, so dass letztlich die gewünschten Restmonomer-Gehalte <0,1% nicht zuverlässig eingehalten wurden.

### Vergleichsbeispiele 2a-e (nicht erfindungsgemäß):

Die Rohprodukte A-E wurden ebenso destilliert wie in den Vergleichsbeispielen 1a-e, mit dem Unterschied, dass als Betriebsmedium für die Kondensatoren nun Kühlsole mit einer Vorlauftemperatur von 4 °C verwendet wurde. Der Druck in den Destillationsstufen betrug anfänglich 25 mbar (1. Stufe), 7 mbar (2. Stufe) und 1,4 mbar (3. Stufe).

In diesem Fällen konnten kurzfristig Restmonomeren-Gehalte <0,1% erreicht werden. Es zeigte sich jedoch in allen Fällen nach wenigen Tagen ein Druckanstieg in den Destillationsapparaten und der gewünschte Restomonomerengehalt <0,1% wurde im Sumpfablauf der 3. Destillationsstufe nicht mehr erreicht. Die Destillation wurde unterbrochen und bei der anschließenden Inspektion der Apparate wurde ein deutlicher Feststoffaufbau auf den Kühlflächen der Kondensatoren festgestellt, der sich durch einfaches Abschmelzen nicht entfernen ließ.

### Beispiele la-e:

Die in den Vergleichsbeispielen 1a-e und Vergleichsbeispielen 2a-e verwendete Destillationsvorrichtung wurde umgebaut, so dass die Brüden jeder Destillationsstufe nun jeweils einen zweiten Kondensator, den Nachkondensator, durchliefen. Die erste Kondensationsstufe wurde jeweils mit Kühlwasser bei einer Vorlauftemperatur von 27 °C betrieben und Nachkondensationsstufe jeweils mit Kühlsole bei einer Vorlauftemperatur von 4 °C. Im Fall des Kurzwegverdampfers wurde also der innenliegende Kondensator mit Kühlwasser und der neu hinzugekommene, externe Nachkondensator mit Kühlsole betrieben.

Die auf diese Weise modifizierte und betriebene Destillationsvorrichtung ermöglichte es, dauerhaft destillierte Sumpfprodukte mit einem Restmonomerengehalt <0,1% herzustellen. Bei Inspektionen der Apparate nach mehrwöchigem Betrieb wurden keine störenden festen Ablagerungen an den Kühlflächen der Kondensatoren festgestellt.

## Patentansprüche

1. Verfahren zur Herstellung von monomerenarmen Polyisocyanaten umfassend die folgenden Schritte:
(i) Modifizierung mindestens eines monomeren Diisocyanats unter Erhalt eines Gemisches enthaltend mindestens ein Polyisocyanat und nicht umgesetztes monomeres Diisocyanat,
(ii) Trennung des in Schritt (i) erhaltenen Gemisches in mindestens einen gasförmigen, monomeres Diisocyanat enthaltenden Strom und einen flüssigen, an monomerem Diisocyanat abgereicherten Strom,
(iii) teilweise Kondensation des gasförmigen Stroms aus (ii) in mindestens einem Kondensator, wobei ein flüssiges Kondensat und ein nicht kondensierter Brüdenstrom erhalten werden,
(iv) Nachkondensation des in Schritt (iii) erhaltenen nicht kondensierten Brüdenstroms in mindestens einem Nachkondensator, wobei ein Nachkondensat und ein nicht kondensiertes Abgas erhalten werden und
(v) Zuführen des nicht kondensierten Abgases aus Schritt (iv) zur Saugseite einer Vakuumpumpe,
**dadurch gekennzeichnet, dass** der mindestens eine Nachkondensator in Schritt (iv) bei einer Nachkondensatortemperatur betrieben wird, und der mindestens eine Kondensator in Schritt (iii) bei einer Kondensatortemperatur betrieben wird, wobei die Nachkondensatortemperatur um ≥ 1 bis ≤ 168 K niedriger liegt als die Kondensatortemperatur.

2. Verfahren gemäß Anspruch 1, **dadurch gekennzeichnet, dass** das monomere Diisocyanat ausgewählt aus der Gruppe bestehend aus 1,5-Diisocyanatopentan (PDI), 1,6-Diisocyanatohexan (HDI), 1-Isocyanato-3,3,5-trimethyl-5-isocyanatomethylcyclohexan (Isophorondiisocyanat, IPDI), 4,4'-Diisocyanatodicyclohexylmethan, 2,4'-Diisocyanatodicyclohexylmethan, Bis-(isocyanatomethyl)-norbornan, 1,3- und 1,4-Bis(isocyanatomethyl)benzol (XDI), Toluylendiisocyanat (TDI), 2,4'- und 4,4'-Diisocyanatodiphenylmethan (MDI) oder beliebige Gemische solcher Diisocyanate.

3. Verfahren gemäß Anspruch 1 oder 2, **dadurch gekennzeichnet, dass** die Modifizierung in Schritt (i) eine intermolekulare Reaktion zwischen Isocyanat-Gruppen, bevorzugt in Gegenwart eines Katalysators, insbesondere in Gegenwart eines basischen Katalysators, unter Bildung von Uretdionpolyisocyanaten, Isocyanuratpolyisocyanaten oder Iminooxadiazindionpolyisocyanaten und/oder eine Reaktion von Isocyanaten mit bereits gebildeten Urethangruppen unter Bildung von Allophanatpolyioscyanaten.

4. Verfahren gemäß einem der Ansprüche 1 bis 3, **dadurch gekennzeichnet, dass** der in Schritt (ii) erhaltene flüssige, an monomerem Diisocyanat abgereicherte Strom höchstens den 0,5-fachen Gehalt, bevorzugt höchstens den 0,1-fachen Gehalt und besonders bevorzugt höchstens den 0,02-fachen Gehalt des monomeren Diisocyanats, bezogen auf den Gehalt in dem in Schritt (i) erhaltenen Gemisch, enthält.

5. Verfahren gemäß einem der Ansprüche 1 bis 4, **dadurch gekennzeichnet, dass** das flüssige Kondensat aus Schritt (iii) zumindest teilweise, gegebenenfalls nach weiteren Reinigungsschritten, in Schritt (i) des Verfahrens, also die Modifizierung des mindestens einen monomeren Diisocyanats, zurückgeführt wird.

6. Verfahren gemäß einem der Ansprüche 1 bis 5, **dadurch gekennzeichnet, dass** die Trennung in Schritt (ii) durch mindestens einen Verdampfer erfolgt, der ausgewählt ist aus der Gruppe bestehend aus Fallfilmverdampfer, Dünnschichtverdampfer oder Kurzwegverdampfer.

7. Verfahren gemäß einem der Ansprüche 1 bis 6, **dadurch gekennzeichnet, dass** die Trennung in Schritt (ii) mehrstufig, vorzugsweise in 2 bis 5 Stufen, besonders bevorzugt in 2 bis 4 Stufen, ganz besonders bevorzugt in 3 bis 4 Stufen und am meisten bevorzugt in 3 Stufen ausgeführt wird.

8. Verfahren gemäß Anspruch 6 oder 7, **dadurch gekennzeichnet, dass** als letzter Verdampfer ein Kurzwegverdampfer eingesetzt wird.

9. Verfahren gemäß einem der Ansprüche 1 bis 8, **dadurch gekennzeichnet, dass** die teilweise Kondensation in Schritt (iii) bei einer Kondensatortemperatur im Bereich von 16 bis 135 °C, bevorzugt im Bereich von 16 bis 40 °C und besonders bevorzugt im Bereich von 18 bis 35 °C erfolgt.

10. Verfahren gemäß einem der Ansprüche 1 bis 9, **dadurch gekennzeichnet, dass** die Nachkondensation in Schritt (iv) bei einer Nachkondensatortemperatur im Bereich von -33 bis 130 °C, bevorzugt im Bereich von -20 bis 25 °C und besonders bevorzugt im Bereich von -10 bis 17 °C erfolgt.

11. Verfahren gemäß einem der Ansprüche 1 bis 10, **dadurch gekennzeichnet, dass** zwischen Schritt (iv) und Schritt (v) ein Schritt (iv-a) durchgeführt wird, in dem der nicht kondensierte Abgasstrom aus dem Nachkondensator in einem weiteren Wärmetauscher, einer Kühlfalle mit einer Kühlfallentemperatur, die mindestens 1 K, bevorzugt mindestens 5 K und besonders bevorzugt mindestens 10 K unterhalb der Nachkondensatortemperatur liegt, weiter abgekühlt wird.

12. Monomerenarmes Polyisocyanat, erhältlich oder erhalten durch ein Verfahren gemäß einem der Ansprüche 1 bis 11.

13. Verwendung eines oder mehrerer monomerenarmer Polyisocyanate gemäß Anspruch 12 als Ausgangsmaterial zur Herstellung von Polymeren wie geschäumten Kunststoffen, Polyurethan-Lacken, Beschichtungsmitteln, Klebstoffen oder Zuschlagstoffen.

14. Polymer, erhältlich oder erhalten durch Umsetzung eines oder mehrerere monomerenarmer Polyisocyanate gemäß Anspruch 12 mit wenigstens einer OH- und/oder NH-Gruppen aufweisenden Komponente.

15. Verbundkörper umfassend wenigstens ein Polymer gemäß Anspruch 14 in direktem Kontakt mit wenigstens einem Substrat bestehend aus Metall, Kunststoff, Holz oder deren Mischungen.
